# EUROPEAN PATENT SPECIFICATION

(11) **EP 4 059 501 B1**
(45) Date of publication and mention of the grant of the patent: **11.11.2026**
(21) Application number: 20891649.4
(22) Date of filing: 26.11.2020
(51) Int. Cl.: A61K 31/4706, A61K 9/16, A61K 9/20, A61P 35/00

(54) **SUBSTITUTED CROTONAMIDE PHARMACEUTICAL COMPOSITION AND PREPARATION METHOD THEREFOR**
SUBSTITUIERTE CROTONAMID-PHARMAZEUTISCHE ZUSAMMENSETZUNG UND HERSTELLUNGSVERFAHREN DAFÜR
COMPOSITION PHARMACEUTIQUE DE CROTONAMIDE SUBSTITUÉ ET SON PROCÉDÉ DE PRÉPARATION

(30) Priority: 27.11.2019 CN 201911180660
(43) Date of publication of application: 21.09.2022
(73) Proprietor: Suzhong Pharmaceutical Group Co., Ltd., Taizhou, Jiangsu 225500 (CN); Jiangsu Suzhong Pharmaceutical Research Institute Co., Ltd., Nanjing, Jiangsu 210000 (CN)
(72) Inventor: TANG, Haitao, Nanjing, Jiangsu 210000 (CN); WANG, Bei, Nanjing, Jiangsu 210000 (CN); GE, Bin, Nanjing, Jiangsu 210000 (CN); GE, Haitao, Nanjing, Jiangsu 210000 (CN); WANG, Zhengjun, Nanjing, Jiangsu 210000 (CN)
(74) Representative: HGF
(86) International application number: PCT/CN2020/131636
(87) International publication number: WO 2021/104340

(56) References cited:
- WO-A2-2010/151710
- CN-A- 101 300 029
- CN-A- 102 625 797
- CN-A- 111 617 081
- CN-A- 111 875 539
- CN-B- 111 617 081

## Description

### TECHNICAL FIELD

The present invention, as defined by the claims, belongs to the field of pharmaceutical preparations, and particularly relates to a substituted crotonamide pharmaceutical composition and a preparation method thereof.

### BACKGROUND

Lung cancer is the malignant tumor with the highest morbidity and mortality in the world, and non-small cell lung cancers (NSCLCs) account for about 80% of all lung cancers. Adenocarcinoma is a non-small cell lung cancer with the highest incidence. At present, driver gene mutations have been found in adenocarcinoma, comprising EGFR, ALK and ROS1 mutations. Compared with chemotherapy drugs used in the past, the survival time of targeted drugs for mutant genes has been significantly improved. The effective rate of the targeted drug therapy for the non-small cell lung cancers with gene mutations is over 70%, the time of tumor control is twice that of chemotherapy, and the side effects are small and the quality of life is high.

Epidermal growth factor receptor tyrosine kinase inhibitors EGFR-TKIs have good therapeutic effects on NSCLC patients with EGFR gene-sensitive mutations. EGFR-TKI treatment is used as a standard treatment in the first line. EGFR-TKIs comprise the first generation of gefitinib, erbtinib and icotinib, the second generation of dacomitinib, afatinib and the third generation of osimertinib. EGFR-TKIs can inhibit tumor growth by blocking EGFR tyrosine kinase phosphorylation activation signal and downstream MAPK and AKT signal pathways in cancer cells, inhibiting proliferation, promoting apoptosis and anti-tumor angiogenesis.

A substituted crotonamide compound (E)-N-(3-cyano-7-ethoxy-4-(3-ethynylphenylamino)quinoline-6-yl)-4-(dimethylamino)butyl-2-enamide or salt thereof is an EGFR-TKI, which is applied to the non-small cell lung cancers. The (E)-N-(3-cyano-7-ethoxy-4-(3-ethynylphenylamino)quinoline-6-yl)-4-(dimethylamino)butyl-2-enamide maleate is also named (E)-N-{4-[(3-ethynylphenylamino)-3-cyano-7-ethoxy -6-quinolinyl]}-4-(dimethylamino)-2-butenamide maleate. A structural formula of the compound is as shown by formula (1):

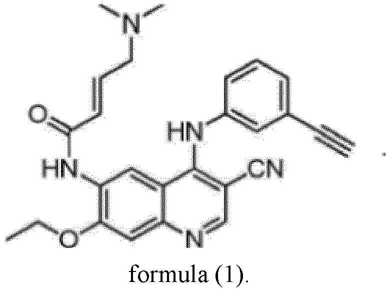

Active pharmaceutical ingredient of the (E)-N-(3-cyano-7-ethoxy-4-(3-ethynylphenylamino)quinoline-6-yl)-4-(dimethylamino)butyl-2-enamide or salt thereof are sensitive to moist heat and produce a related impurity A as shown by structural formula (2) under moist heat conditions.

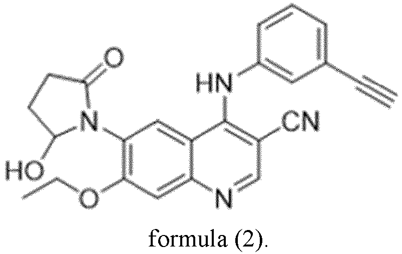

No prior art has been documented to control or inhibit the production of the impurity A of the (E)-N-(3-cyano-7-ethoxy-4-(3-ethynylphenylamino)quinoline-6-yl)-4-(dimethylamino)butyl-2-enamide or salt by means of preparation compositions.

International patent publication No. WO2010151710A2 discloses quinolines and quniazolines, their derivatives, pharmaceutically acceptable salts, solvates and hydrates thereof which are used for the treatment of protein kinases mediated diseases and conditions.

### SUMMARY

The technical problem to be solved by the present invention is to provide a pharmaceutical composition of a substituted crotonamide compound (E)-N-(3-cyano-7-ethoxy-4-(3-ethynylphenylamino)quinoline-6-yl)-4-(dimethylamino)butyl-2-enamide or salt thereof, as defined by the claims. The pharmaceutical composition has good stability.

An aspect of the present invention provides a pharmaceutical composition of (E)-N-(3-cyano-7-ethoxy-4-(3-ethynylphenylamino)quinoline-6-yl)-4-(dimethylamino)butyl-2-enamide or salt thereof, comprising the following ingredients in parts by weight: 5 parts to 50 parts of (E)-N-(3-cyano-7-ethoxy-4-(3-ethynylphenylamino)quinoline-6-yl)-4-(dimethylamino)butyl-2-enamide or salt thereof, 40 parts to 120 parts of filler, 2 parts to 20 parts of disintegrant, 0 part to 6 parts of adhesive, and 0.5 part to 5 parts of lubricant, as defined by the claims.

The ingredients are further 8 parts to 12 parts of (E)-N-(3-cyano-7-ethoxy-4-(3-ethynylphenylamino)quinoline-6-yl)-4-(dimethylamino)butyl-2-enamide or salt thereof, 50 parts to 100 parts of filler, 4 parts to 15 parts of disintegrant, 0.3 part to 5 parts of adhesive, and 0.5 part to 5 parts of lubricant. More further, the ingredients are 9 parts to 11 parts of (E)-N-(3-cyano-7-ethoxy-4-(3-ethynylphenylamino)quinoline-6-yl)-4-(dimethylamino)butyl-2-enamide or salt thereof, 65 parts to 90 parts of filler, 5 parts to 12 parts of disintegrant, 0.5 part to 3 parts of adhesive, and 0.5 part to 4 parts of lubricant. More specifically, the ingredients are 10 parts of (E)-N-(3-cyano-7-ethoxy-4-(3-ethynylphenylamino)quinoline-6-yl)-4-(dimethylamino)butyl-2-enamide or salt thereof, 65 parts to 90 parts of filler, 5 parts to 12 parts of disintegrant, 0.5 part to 3 parts of adhesive, and 0.5 part to 4 parts of lubricant.

The filler is selected from a carbohydrate, is preferably a compound of saccharide, and is more preferably sugar alcohol. The sugar alcohol is selected from one or more of mannitol, xylitol, sorbitol and lactose, and is more preferably one or more of mannitol and lactose.

The disintegrant is sodium carboxymethyl starch.

The adhesive is one or two of hydroxypropyl cellulose or hydroxypropyl methyl cellulose, and is preferably hydroxypropyl cellulose.

The lubricant is glyceryl behenate.

The salt of the (E)-N-(3-cyano-7-ethoxy-4-(3-ethynylphenylamino)quinoline-6-yl)-4-(dimethylamino)butyl-2-enamide is a pharmaceutically acceptable salt.

The pharmaceutically acceptable salts comprises those salts commonly used to form alkali metal salts and free acid or free base addition salts approved by regulatory agencies. Salts are formed by ion association, charge-charge interaction, covalent bonding, complextion and coordination. As long as the salts are pharmaceutically acceptable, the properties of the salts are not critical.

Types of the pharmaceutically acceptable salt comprise but are not limited, acid addition salts formed by reacting a free alkali form of the compound with the following pharmaceutically acceptable acids: inorganic acids such as a hydrochloric acid, a hydrobromic acid, a sulfuric acid, a phosphoric acid, or the like; or organic acids such as an acetic acid, a propionic acid, a glycolic acid, a pyruvic acid, a lactic acid, a malonic acid, a succinic acid, a malic acid, a maleic acid, a fumaric acid, a tartaric acid, a citric acid, a methanesulfonic acid, a benzenesulfonic acid, a toluenesulfonic acid, a gluconic acid, a glutamic acid, a naphtholcarboxylic acid, a salicylic acid, or the like.

Other examples of such salts may be found in J. Pharm. Sci, 66, 1(1977) written by Berge et al. In some embodiments, the salt is formed using conventional methods. For example, the phosphate of the compound according to the present invention is prepared by mixing a desired solvent or a desired compound free alkali in a solvent combination with a desired stoichiometric amount of phosphoric acid at a desired temperature, usually under heating (depending on a boiling point of the solvent). In one embodiment, after (slow or rapid) cooling, the salt is precipitated and crystallized (i.e., if the salt has crystalline properties). Moreover, semi-salt, mono-salt, di-salt, tri-salt and multi-salt forms of the compounds of the present invention are also comprised herein. Similarly, the compound, and the salt or semihydrate, monohydrate, dihydrate, trihydrate and polyhydrate forms thereof are also comprised herein.

In some embodiments, the compound is a hydrochloride, a hydrobromide, a sulfate, a phosphate or metaphosphate, an acetate, a propionate, a caproate, a cyclopentane propionate, a glycollate, a pyruvate, a lactate, a malonate, a succinate, a malate, a maleate, a fumarate, a trifluoroacetate, a tartarate, a citrate, a benzoate, a 3-(4-hydroxybenzoyl)benzoate, a cinnamate, a mandelate, a mesylate, an esilate, 1,2-ethanesulfonate, 2-hydroxyethanesulfonate, a benzene sulfonate, a toluene sulfonate, a 2-naphthalenesulfonate, a 4-methylbicyclo-[2.2.2]oct-2-ene-1-carboxylate, a glucosinolate, a 4'4-methylene-bis-(3-hydroxy-2-ene-1-carboxylate)salt, a 3-phenylpropionate, a pivalate, a tert-butyl acetate, a lauryl sulfate, a gluconate, a glutamate, a hydroxynaphthoate, a salicylate, a stearate, a muconate, a butyrate, a phenylacetate, a phenylbutyrate, a valproate, and the like.

In a preferred embodiment, the salt of the compound is a hydrochloride, a benzene sulfonate, a mesylate, a maleate, or a hydrate thereof, such as a monohydrate. Specifically, the (E)-N-(3-cyano-7-ethoxy-4-(3-ethynylphenylamino)quinoline-6-yl)-4-(dimethylamino)butyl-2-enamide maleate and a maleate monohydrate thereof are particularly suitable.

A moisture content of the pharmaceutical composition according to the present invention is preferably within 5wt%, further preferably within 3wt%, and more preferably within 2wt% or 1.5wt%, and the moisture content within 1wt% is particularly suitable as well.

The pharmaceutical composition according to the present invention, as defined by the claims, also further comprises a pharmaceutically acceptable adjuvant, comprising but not limited to a carrier, an excipient, an adhesive, a filler, a suspending agent, an aromatic, a sweeteners, a disintegrant, a dispersant, a surfactant, a lubricant, a colorant, a diluent, a solubilizer, a humectant, a plasticizer, a stabilizer, a penetration enhancer, a wetting agent, a defoamer, an antioxidant, a preservative, or one or more of these adjuvants. The pharmaceutical composition facilitates the administration of the compound to an organism.

The pharmaceutical composition of the present invention may be further made into pharmaceutical preparations to facilitate administration to patents. The pharmaceutical preparations comprise but are not limited to an aqueous liquid dispersion, a self-emulsifying dispersion, a solid solution, a liposome dispersion, an aerosol, a solid dosage form, a powder, a quick-release preparation, a controlled-release preparation, a disintegrating (fastmelt) preparation, a tablet, a capsule, a pill, a delayed-release preparation, an extended-release preparation, a pulse-release preparation, multiple granule preparations and mixed quick-release and controlled-release preparations.

Another aspect of the present invention provides a preparation method of the pharmaceutical composition of (E)-N-(3-cyano-7-ethoxy-4-(3-ethynylphenylamino)quinoline-6-yl)-4-(dimethylamino)butyl-2-enamide or salt thereof, comprising the following steps of:
(1) pre-mixing: evenly mixing the (E)-N-(3-cyano-7-ethoxy-4-(3-ethynylphenylamino)quinoline-6-yl)-4-(dimethylamino)butyl-2-enamide or salt thereof, the filler and the disintegrant;
(2) wet-granulating: adding an adhesive solution to prepare a soft material, and then sieving and granulating the soft material to prepare wet granules; and
(3) drying, grading and blending: drying, sieving and grading the wet granules, and adding the lubricant for blending to prepare the pharmaceutical composition. The pharmaceutical composition may be further pressed into tablets or encapsulated into capsules. The pre-mixing mode in step (1) comprises airflow pulverization pre-mixing, sieving pre-mixing and wet granulator pre-mixing; the adhesive in step (2) is prepared from purified water with a concentration of 2wt% to 10wt%; the wet-granulating in step (2) may be carried out by using a wet granulator or a fluidized bed, and granulating and drying by a fluidized bed is preferable; and the drying in step (3) may be carried out by forced air drying or fluidized bed, and preferably carried out by fluidized bed; and the moisture content of the dried granules are controlled to be within 1.5wt%, and preferably within 1wt%.

The present invention provides another preparation method of the pharmaceutical composition of (E)-N-(3-cyano-7-ethoxy-4-(3-ethynylphenylamino)quinoline-6-yl)-4-(dimethylamino)butyl-2-enamide or salt thereof, comprising the following steps of:
(1) pre-mixing: evenly mixing active ingredients, the filler and the disintegrant; and
(2) drying and blending: adding the lubricant into the dried powder for blending. The pre-mixing mode in step (1) comprises airflow pulverization pre-mixing, sieving pre-mixing and wet-granulator pre-mixing; the drying in step (2) may be carried out by forced air drying, and a moisture content after drying is controlled to be within 3wt% or 1.5wt%, and preferably within 1wt%.

Specifically, in the preparation process of the pharmaceutical composition of the present invention, the maleic acid (E)-N-(3-cyano-7-ethoxy-4-(3-ethynylphenylamino)quinoline-6-yl)-4-(dimethylamino)butyl-2-enamide or salt thereof is evenly mixed with the filler and the disintegrant, and then the adhesive solution is added to prepare a soft material, then the soft material is granulated and dried, the moisture content of the dried granules is controlled to be within 3wt% or 1.5wt%, and finally the lubricant is added for blending to prepare the pharmaceutical composition. The pharmaceutical composition may be added with other pharmaceutically acceptable adjuvants to prepare pharmaceutical preparations such as tablets or capsules. Alternatively, the active ingredient maleic acid (E)-N-(3-cyano-7-ethoxy-4-(3-ethynylphenylamino)quinoline-6-yl)-4-(dimethylamino)butyl-2-enamide or salt thereof is evenly mixed with the filler and the disintegrant, and then the lubricant is added for blending and drying, and the moisture content of the dried granules is controlled to be within 3wt% or 1.5wt% to prepare the pharmaceutical composition. The pharmaceutical composition may be added with other pharmaceutically acceptable adjuvants to prepare pharmaceutical preparations such as tablets or capsules.

More particularly, a preferred preparation method of the pharmaceutical composition of the present invention comprises the following steps of:
(1) pre-mixing: evenly mixing the active ingredient, the filler and the disintegrant;
(2) wet-granulating: adding an adhesive solution to prepare a soft material, and then sieving the soft material with a sieve of 20 meshes to 30 meshes for granulating; and
(3) drying, grading and blending: drying the wet granules, then sieving and grading the wet granules with sieve of 20 meshes to 30 meshes for grading, and adding the lubricant for blending to prepare the pharmaceutical composition.

The method for detecting the impurity A in the present invention is as follows:
instrument: Agilent 1260 high performance liquid chromatograph, and chromatographic column: Agilent ZORBAX Extend (4.6 mm×250 mm, 5 µm), mobile phase: 1wt% ammonium acetate solution (adjusted to a pH of 7.0 with triethylamine or acetic acid) as a mobile phase A, and acetonitrile as a mobile phase B; a gradient elution procedure is shown in Table 1. Detection wavelength: 261 nm, flow rate: 1.0 mL/min, column temperature: 25°C, sample size: 10 µL, solution preparation: diluent: acetonitrile-water (v/v of 60: 40).

**Table 1 Gradient elution parameters of mobile phase**

| Time (min) | Mobile phase A (%) | Mobile phase B (%) |
|---|---|---|
| 0 | 39 | 61 |
| 25 | 39 | 61 |
| 50 | 70 | 30 |
| 50.01 | 39 | 61 |
| 60 | Stop | |

The present invention has the beneficial effects that:
By adopting the pharmaceutical composition of the present invention, a fluidity of materials is greatly improved, and problems of difficult preparation, low content of finished products and increased content of the impurity A caused by easy adhesion of raw materials to container walls due to high viscosity of the raw materials are solved. Meanwhile, the pharmaceutical composition has good quality stability.

### DETAILED DESCRIPTION (only those compositions according to the claims are according to the invention, all other compositions relate to comparative examples)

A pharmaceutical composition of (E)-N-(3-cyano-7-ethoxy-4-(3-ethynylphenylamino)quinoline-6-yl)-4-(dimethylamino)butyl-2-enamide maleate monohydrate (hereinafter referred to as active ingredient) and a preparation method are further illustrated by the following embodiments. However, these embodiments do not constitute any limitation to the present invention. The active ingredient is prepared according to the method described in CN104513200A.

### Embodiment 1

Table 2 shows single dose prescription compositions.

**Table 2**

| Ingredient | Single dose (mg) |
|---|---|
| Active ingredient | 20 |
| Mannitol | 120 |
| Microcrystalline cellulose | 35 |
| Polyvinylpolypyrrolidone | 7 |
| Polyvidone K30 | 5 |
| Magnesium stearate | 1 |
| Colloidal silicon dioxide | 5 |
| Total | 193 |

The prescription amounts of active ingredient, mannitol, microcrystalline cellulose and polyvinylpolypyrrolidone were added into a wet granulator and mixed evenly to prepare a soft material by using 15wt% polyvidone K30 as an adhesive, then the soft material was granulated by a sieve of 20 meshes and dried, and a moisture content of the granules was controlled to be within 1.5wt%. After grading by 10 meshes, the prescription amounts of magnesium stearate and colloidal silicon dioxide were added in the granules. After blending, intermediate granules were encapsulated into capsules.

### Embodiment 2

Table 3 shows single dose prescription compositions.

**Table 3**

| Ingredient | Single dose (mg) |
|---|---|
| Active ingredient | 20 |
| Microcrystalline | 170 |
| Polyvidone K30 | 10 |
| Glyceryl behenate | 4 |
| Colloidal silicon dioxide | 2 |
| Total | 206 |

The prescription amounts of active ingredient and microcrystalline cellulose were added into a wet granulator and mixed evenly to prepare a soft material by using 15wt% polyvidone K30 as an adhesive, then the soft material was granulated by a sieve of 20 meshes and dried, and a moisture content of the granules was controlled to be within 1.5wt%. After grading by 0 mesh to 30 meshes, the prescription amounts of glyceryl behenate and colloidal silicon dioxide were added in the granules. After blending, intermediate granules were encapsulated into capsules.

### Embodiment 3

Table 4 shows single dose prescription compositions.

**Table 4**

| Ingredient | Single dose (mg) |
|---|---|
| Active ingredient | 20 |
| Mannitol | 170 |
| Polyvidone K30 | 10 |
| Glyceryl behenate | 4 |
| Colloidal silicon dioxide | 2 |
| Total | 206 |

The prescription amounts of active ingredient and mannitol were added into a wet granulator and mixed evenly to prepare a soft material by using 15wt% polyvidone K30 as an adhesive, then the soft material was granulated by a sieve of 20 meshes and dried, and a moisture content of the granules was controlled to be within 1.5wt%. After grading, the prescription amounts of glyceryl behenate and colloidal silicon dioxide were added in the granules. After blending, intermediate granules were encapsulated into capsules.

### Embodiment 4

Table 5 shows single dose prescription compositions.

**Table 5**

| Ingredient | Single dose (mg) |
|---|---|
| Active ingredient | 20 |
| Mannitol | 160 |
| Sodium carboxymethyl starch | 10 |
| Hydroxypropyl cellulose | 1 |
| Glyceryl behenate | 4 |
| Total | 195 |

The prescription amounts of active ingredient and sodium carboxymethyl starch were added into a wet granulator and mixed evenly to prepare a soft material by using 4wt% hydroxypropyl cellulose as an adhesive, then the soft material was granulated by a sieve of 20 meshes and dried, and a moisture content of the granules was controlled to be within 1.5wt%. After grading by 10 meshes, the prescription amounts of glyceryl behenate were added in the granules. After blending, intermediate granules were encapsulated into capsules.

The capsule granules of Embodiments 1, 2, 3 and 4 were placed in open weighing bottles at a high temperature of 40°C for one month and then taken out to determine contents of related substances (%). The results were shown in Table 6.

**Table 6**

| Time | Embodiment 1 | Embodiment 2 | Embodiment 3 | Embodiment 4 |
|---|---|---|---|---|
| 0 day | 0.08 | 0.07 | 0.05 | 0.04 |
| 14 days | 0.15 | 0.32 | 0.12 | 0.08 |
| 1 month | 0.24 | 0.46 | 0.26 | 0.14 |

The above results show that: After being placed at high temperature for one month, the related substance impurity A of the composition of Embodiment 4 increases slowly. This indicates that the stability of the pharmaceutical composition prepared according to the technical solution of the present invention is significantly improved compared with other prescription compositions.

### Embodiment 5

Table 7 shows single dose prescription compositions.

**Table 7**

| Ingredient | Single dose (mg) |
|---|---|
| Active ingredient | 20 |
| Mannitol | 200 |
| Sodium carboxymethyl starch | 10 |
| Hydroxypropyl cellulose | 3 |
| Glyceryl behenate | 5 |
| Total | 238 |

The prescription amounts of active ingredient, mannitol and sodium carboxymethyl starch were added into a wet granulator and mixed evenly to prepare a soft material by using 4wt% hydroxypropyl cellulose as an adhesive, then the soft material was granulated by a sieve of 20 meshes and dried to prepare granules with moisture contents 0.8wt%, 1.0wt% and 1.4wt% respectively. After grading, the prescription amounts of glyceryl behenate were added in each granule respectively. After blending, intermediate granules were encapsulated into capsules.

The capsule granules of Embodiment 5 with different moisture contents were placed for 14 days under the conditions of high temperature of 40°C, high humidity of 92.5% RH, illumination of 4,500 lux and acceleration (high temperature of 40°C and high humidity of 75%) respectively, and then taken out to determine the contents (%) of the related substance impurity. The results were shown in Table 8.

**Table 8**

| Related substance (%) | Granules with a moisture content of 0.8wt% | Granules with a moisture content of 1.0wt% | Granules with a moisture content of 1.4wt% |
|---|---|---|---|
| 0 days | 0.03 | 0.03 | 0.04 |
| High temperature for 14 days | 0.09 | 0.10 | 0.10 |
| High humidity for 14 days | 0.08 | 0.07 | 0.08 |
| Illumination for 14 days | 0.07 | 0.08 | 0.08 |
| Acceleration for 14 days | 0.09 | 0.08 | 0.09 |

The above results show that: The preparation composition of granules with a moisture content within 1.5wt% is relatively stable.

### Embodiment 6

Table 9 shows single dose prescription compositions.

**Table 9**

| Ingredient | Single dose (mg) |
|---|---|
| Active ingredient | 20 |
| Mannitol | 160 |
| Polyvinylpolypyrrolidone | 10 |
| Hydroxypropyl cellulose | 1 |
| Glyceryl behenate | 4 |
| Total | 195 |

The prescription amounts of active ingredient, mannitol and polyvinylpolypyrrolidone were added into a wet granulator and mixed evenly to prepare a soft material by using 4wt% hydroxypropyl cellulose as an adhesive, then the soft material was granulated by a sieve of 20 meshes and dried, and a moisture content of the granules was controlled to be within 1.5wt%. After grading, the prescription amounts of glyceryl behenate were added in the granules. After blending, intermediate granules were encapsulated into capsules.

### Embodiment 7

Table 10 shows single dose prescription compositions.

**Table 10**

| Ingredient | Single dose (mg) |
|---|---|
| Active ingredient | 20 |
| Mannitol | 130 |
| Sodium carboxymethyl starch | 20 |
| Hydroxypropyl cellulose | 1 |
| Glyceryl behenate | 1 |
| Total | 172 |

The prescription amounts of active ingredient, mannitol and sodium carboxymethyl starch were added into a wet granulator and mixed evenly to prepare a soft material by using 4wt% hydroxypropyl cellulose as an adhesive, then the soft material was granulated by a sieve of 20 meshes and dried, and a moisture content of the granules was controlled to be within 1.5wt%. The prescription amounts of glyceryl behenate were added. After blending, intermediate granules were encapsulated into capsules.

### Embodiment 8

Table 11 shows single dose prescription compositions.

**Table 11**

| Ingredient | Single dose (mg) |
|---|---|
| Active ingredient | 20 |
| lactose monohydrate | 160 |
| Sodium | 10 |
| Hydroxypropyl | 1.5 |
| Glyceryl behenate | 8 |
| Total | 199.5 |

The prescription amounts of active ingredient, lactose monohydrate and sodium carboxymethyl starch were added into a wet granulator and mixed evenly to prepare a soft material by using 4wt% hydroxypropyl cellulose as an adhesive, then the soft material was granulated by a sieve of 20 meshes for granulating and dried, and a moisture content of the granules was controlled to be within 1.5wt%. After grading, the prescription amounts of glyceryl behenate were added in the granules. After blending, intermediate granules were encapsulated into capsules.

The capsules of Embodiments 1, 2, 3, 4, 6, 7 and 8 were respectively packed in high-density polyethylene bottles, with 20 capsules per bottle, and 2 bags/bottle of desiccant were added. The bottles were placed under acceleration (high temperature of 40°C and high humidity of 75%) conditions for 3 months. The content (%) change results of the impurities A in each batch were shown in Table 12.

**Table 12**

| Impurity A (%) | Embodiment 1 | Embodiment 4 | Embodiment 6 | Embodiment 7 | Embodiment 8 |
|---|---|---|---|---|---|
| 0 day | 0.08 | 0.04 | 0.09 | 0.05 | 0.06 |
| Acceleration for 1 month | 0.28 | 0.14 | 0.15 | 0.15 | 0.12 |
| Acceleration for 2 months | 0.48 | 0.20 | 0.25 | 0.21 | 0.18 |
| Acceleration for 3 month3 | 0.62 | 0.20 | 0.47 | 0.22 | 0.20 |

The above results show that: the prescription stabilities of Embodiments 4, 7 and 8 are significantly higher than that of other prescriptions after being placed for 3 months under acceleration conditions, which indicates that the stability of the capsules prepared according to the technical solution of the present invention is significantly improved.

## Claims

1. A pharmaceutical composition of (E)-N-(3-cyano-7-ethoxy-4-(3-ethynylphenylamino)quinolin-6-yl)-4-(dimethylamino)but-2-enamid e or salt thereof, comprising the following ingredients in parts by weight: 5 parts to 50 parts of the (E)-N-(3-cyano-7-ethoxy-4-(3-ethynylphenylamino)quinolin-6-yl)-4-(dimethylamino)but-2-enamid e or salt thereof, 40 parts to 120 parts of filler, 2 parts to 20 parts of disintegrant, 0 part to 6 parts of adhesive, and 0.5 part to 5 parts of lubricant, wherein the disintegrant is sodium carboxymethyl starch and the lubricant is glyceryl behenate.

2. The pharmaceutical composition according to claim 1, comprising the following ingredients in parts by weight: 8 parts to 12 parts of (E)-N-(3-cyano-7-ethoxy-4-(3-ethynylphenylamino)quinoline-6-yl)-4-(dimethylamino)butyl-2-ena mide or salt thereof, 50 parts to 100 parts of filler, 4 parts to 15 parts of disintegrant, 0.3 part to 5 parts of adhesive, and 0.5 part to 5 parts of lubricant.

3. The pharmaceutical composition according to claim 1, comprising the following ingredients in parts by weight: 9 parts to 11 parts of (E)-N-(3-cyano-7-ethoxy-4-(3-ethynylphenylamino)quinoline-6-yl)-4-(dimethylamino)butyl-2-ena mide or salt thereof, 65 parts to 90 parts of filler, 5 parts to 12 parts of disintegrant, 0.5 part to 3 parts of adhesive, and 0.5 part to 4 parts of lubricant.

4. The pharmaceutical composition according to any one of claims 1 to 3, wherein the filler is selected from a carbohydrate, is preferably a compound of saccharide, and is more preferably sugar alcohol.

5. The pharmaceutical composition according to claim 4, wherein the sugar alcohol is one or more of mannitol, xylitol, sorbitol and lactose, and is more preferably one or more of mannitol and lactose.

6. The pharmaceutical composition according to any one of claims 1 to 3, wherein the adhesive is one of hydroxypropyl cellulose or hydroxypropyl methyl cellulose, and is preferably hydroxypropyl cellulose.

7. The pharmaceutical composition according to any one of claims 1 to 3, wherein the salt is hydrochloride, benzene sulfonate, mesylate or maleate, is further a hemihydrate or a monohydrate of hydrochloride, benzene sulfonate, mesylate or maleate, and is preferably the monohydrate of maleate; and preferably, a moisture content of the pharmaceutical composition is within 5wt% or 3wt% or 1.5wt%.

8. A preparation method of the pharmaceutical composition according to any one of claims 1 to 7, comprising the following steps of:
(1) pre-mixing: evenly mixing the (E)-N-(3-cyano-7-ethoxy-4-(3-ethynylphenylamino)quinoline-6-yl)-4-(dimethylamino)butyl-2-ena mide maleate, the filler and the disintegrant;
(2) wet-granulating: adding an adhesive solution to prepare a soft material, and then sieving and granulating the soft material to prepare wet granules; and
(3) drying, grading and blending: drying, sieving and grading the wet granules, and adding the lubricant for blending to prepare the pharmaceutical composition.

9. The preparation method of the pharmaceutical composition according to claim 8, wherein the adhesive in step (2) is prepared from purified water, with a concentration of 2wt% to 10wt%; and a moisture content of the dried granules in step (3) is controlled within 1.5wt%, and is preferably controlled within 1wt%.

## Patentansprüche

1. Pharmazeutische Zusammensetzung von (E)-N-(3-Cyano-7-ethoxy-4-(3-ethynylphenylamino)chinolin-6-yl)-4-(dimethylamino)but-2-enamid e oder Salz davon, umfassend die folgenden Inhaltsstoffe in Gewichtsteilen: 5 Teile bis 50 Teile des (E)-N-(3-Cyano-7-ethoxy-4-(3-ethynylphenylamino)chinolin-6-yl)-4-(dimethylamino)but-2-enamid e oder Salz davon, 40 Teile bis 120 Teile Füllstoff, 2 Teile bis 20 Teile Sprengmittel, 0 Teile bis 6 Teile Klebstoff und 0,5 Teile bis 5 Teile Schmiermittel, wobei das Sprengmittel Natriumcarboxymethylstärke ist und das Schmiermittel Glycerylbehenat ist.

2. Pharmazeutische Zusammensetzung nach Anspruch 1, umfassend die folgenden Inhaltsstoffe in Gewichtsteilen: 8 Teile bis 12 Teile (E)-N-(3-Cyano-7-ethoxy-4-(3-ethynylphenylamino)chinolin-6-yl)-4-(dimethylamino)butyl-2-enamid oder Salz davon, 50 Teile bis 100 Teile Füllstoff, 4 Teile bis 15 Teile Sprengmittel, 0,3 Teile bis 5 Teile Klebstoff und 0,5 Teile bis 5 Teile Schmiermittel.

3. Pharmazeutische Zusammensetzung nach Anspruch 1, umfassend die folgenden Inhaltsstoffe in Gewichtsteilen: 9 Teile bis 11 Teile (E)-N-(3-Cyano-7-ethoxy-4-(3-ethynylphenylamino)chinolin-6-yl)-4-(dimethylamino)butyl-2-enamid oder Salz davon, 65 Teile bis 90 Teile Füllstoff, 5 Teile bis 12 Teile Sprengmittel, 0,5 Teile bis 3 Teile Klebstoff und 0,5 Teile bis 4 Teile Schmiermittel.

4. Pharmazeutische Zusammensetzung nach einem der Ansprüche 1 bis 3, wobei der Füllstoff aus einem Kohlenhydrat ausgewählt ist, bevorzugt eine Verbindung von Saccharid ist und bevorzugter Zuckeralkohol ist.

5. Pharmazeutische Zusammensetzung nach Anspruch 4, wobei der Zuckeralkohol eines oder mehrere von Mannit, Xylit, Sorbit und Lactose ist und bevorzugter eines oder mehrere von Mannit und Lactose ist.

6. Pharmazeutische Zusammensetzung nach einem der Ansprüche 1 bis 3, wobei der Klebstoff eines von Hydroxypropylcellulose oder Hydroxypropylmethylcellulose ist und bevorzugt Hydroxypropylcellulose ist.

7. Pharmazeutische Zusammensetzung nach einem der Ansprüche 1 bis 3, wobei das Salz Hydrochlorid, Benzolsulfonat, Mesylat oder Maleat ist, ferner ein Hemihydrat oder ein Monohydrat von Hydrochlorid, Benzolsulfonat, Mesylat oder Maleat ist und bevorzugt das Monohydrat von Maleat ist; und bevorzugt ein Feuchtigkeitsgehalt der pharmazeutischen Zusammensetzung innerhalb von 5 Gew.-% oder 3 Gew.-% oder 1,5 Gew.-% liegt.

8. Herstellungsverfahren der pharmazeutischen Zusammensetzung nach einem der Ansprüche 1 bis 7, umfassend die folgenden Schritte:
(1) Vormischen: Gleichmäßiges Mischen des (E)-N-(3-Cyano-7-ethoxy-4-(3-ethynylphenylamino)chinolin-6-yl)-4-(dimethylamino)butyl-2- enamidmaleats, des Füllstoffs und des Sprengmittels;
(2) Nassgranulieren: Hinzufügen einer Klebstofflösung, um ein weiches Material herzustellen, und dann Sieben und Granulieren des weichen Materials, um nasse Granulate herzustellen; und
(3) Trocknen, Sortieren und Mischen: Trocknen, Sieben und Sortieren des feuchten Granulats und Hinzufügen des Schmiermittels zum Vermischen, um die pharmazeutische Zusammensetzung herzustellen.

9. Herstellungsverfahren der pharmazeutischen Zusammensetzung nach Anspruch 8, wobei der Klebstoff in Schritt (2) aus gereinigtem Wasser mit einer Konzentration von 2 Gew.-% bis 10 Gew.-% hergestellt wird; und ein Feuchtigkeitsgehalt des getrockneten Granulats in Schritt (3) innerhalb von 1,5 Gew.-% gesteuert wird und bevorzugt innerhalb von 1 Gew.-% gesteuert wird.

## Revendications

1. Composition pharmaceutique de (E)-N-(3-cyano-7-éthoxy-4-(3-éthynylphénylamino)quinolin-6-yl)-4-(diméthylamino)but-2-énamide ou d'un sel de celui-ci, comprenant les ingrédients suivants en parties en poids : 5 parties à 50 parties du (E)-N-(3-cyano-7-éthoxy-4-(3-éthynylphénylamino)quinolin-6-yl)-4-(diméthylamino)but-2-énamide ou d'un sel de celui-ci, 40 parties à 120 parties d'une charge, 2 parties à 20 parties d'un désintégrant, 0 partie à 6 parties d'un adhésif et 0,5 partie à 5 parties d'un lubrifiant, dans laquelle le désintégrant est le carboxyméthylamidon sodique et le lubrifiant est le béhénate de glycéryle.

2. Composition pharmaceutique selon la revendication 1, comprenant les ingrédients suivants en parties en poids : 8 parties à 12 parties de (E)-N-(3-cyano-7-éthoxy-4-(3-éthynylphénylamino)quinolin-6-yl)-4-(diméthylamino)butyl-2-énamide ou d'un sel de celui-ci, 50 parties à 100 parties d'une charge, 4 parties à 15 parties d'un désintégrant, 0,3 partie à 5 parties d'un adhésif et 0,5 partie à 5 parties d'un lubrifiant.

3. Composition pharmaceutique selon la revendication 1, comprenant les ingrédients suivants en parties en poids : 9 parties à 11 parties de (E)-N-(3-cyano-7-éthoxy-4-(3-éthynylphénylamino)quinolin-6-yl)-4-(diméthylamino)butyl-2-énamide ou d'un sel de celui-ci, 65 parties à 90 parties d'une charge, 5 parties à 12 parties d'un désintégrant, 0,5 partie à 3 parties d'un adhésif et 0,5 partie à 4 parties d'un lubrifiant.

4. Composition pharmaceutique selon l'une quelconque des revendications 1 à 3, dans laquelle la charge est choisie parmi un glucide, est de préférence un composé saccharidique, et est idéalement un alcool glucidique.

5. Composition pharmaceutique selon la revendication 4, dans laquelle l'alcool glucidique est l'un ou plusieurs du mannitol, du xylitol, du sorbitol et du lactose, et est idéalement l'un ou plusieurs du mannitol et du lactose.

6. Composition pharmaceutique selon l'une quelconque des revendications 1 à 3, dans laquelle l'adhésif est l'une de l'hydroxypropylcellulose ou de l'hydroxypropylméthylcellulose, et est de préférence l'hydroxypropylcellulose.

7. Composition pharmaceutique selon l'une quelconque des revendications 1 à 3, dans laquelle le sel est un chlorhydrate, un benzènesulfonate, un mésylate ou un maléate, est en outre un hémihydrate ou un monohydrate de chlorhydrate, de benzènesulfonate, de mésylate ou de maléate, et est de préférence le monohydrate de maléate ; et de préférence, une teneur en humidité de la composition pharmaceutique est d'au plus 5 % en poids ou 3 % en poids ou 1,5 % en poids.

8. Procédé de préparation de la composition pharmaceutique selon l'une quelconque des revendications 1 à 7, comprenant les étapes suivantes de :
(1) prémélange : mélange uniforme du maléate de (E)-N-(3-cyano-7-éthoxy-4-(3-éthynylphénylamino)quinolin-6-yl)-4-(diméthylamino)butyl-2-énamide, de la charge et du désintégrant ;
(2) granulation humide : ajout d'une solution d'adhésif pour préparer un matériau mou, puis tamisage et granulation du matériau mou pour préparer des granulés humides ; et
(3) séchage, calibrage et mélange : séchage, tamisage et calibrage des granulés humides, et ajout du lubrifiant pour le mélange afin de préparer la composition pharmaceutique.

9. Procédé de préparation de la composition pharmaceutique selon la revendication 8, dans lequel l'adhésif de l'étape (2) est préparé à partir d'eau purifiée, à une concentration de 2 % en poids à 10 % en poids ; et une teneur en humidité des granulés séchés à l'étape (3) est contrôlée à 1,5 % en poids au maximum, et est de préférence contrôlée à 1 % en poids au maximum.
